# EUROPEAN PATENT SPECIFICATION

(11) **EP 1 095 059 B1**
(45) Date of publication and mention of the grant of the patent: **07.06.2006**
(21) Application number: 99933284.4
(22) Date of filing: 12.07.1999
(51) Int. Cl.: C07K 14/31, C07K 16/12, A61K 38/00, A61K 39/085, A61K 39/40, A61P 31/04, A61P 31/18, A61P 37/02, G01N 33/68

(54) **CHEMOTAXIS INHIBITING PROTEIN OF STAPHYLOCOCCUS (CHIPS) AND ITS USE**
CHEMOTAXIS HEMMENDES PROTEIN VON STAPHYLOCOCCUS AUREUS (CHIPS) UND DESSEN VERWENDUNG
PROTEINE DU $i(STAPHYLOCOQUE) INHIBITRICE DE CHIMIOTACTISME ET SON UTILISATION

(30) Priority: 10.07.1998 NL 1009614
(43) Date of publication of application: 02.05.2001
(73) Proprietor: Alligator Bioscience AB (publ), 223 70 Lund (SE)
(72) Inventor: VAN STRIJP, Johannes, Antonius, Gerardus, 3984 NV Odijk (NL); VAN KESSEL, Cornelis, Petrus, Maria, NL-3981 EX Bunnik (NL)
(74) Representative: Thomas, Philip John Duval
(86) International application number: PCT/NL1999/000442
(87) International publication number: WO 2000/002913

(56) References cited:
- WO-A-87/07146
- WO-A-93/04202
- WO-A-96/40907
- US-A- 5 514 555
- VELDKAMP ET AL.: "Specific downregulation of neutrophil chemotaxis by staphylococcal culture supernates" ABSTRACTS OF THE GENERAL MEETING OF THE AMERICAN SOCIETY FOR MICROBIOLOGY, vol. 97, 1997 - 8 May 1998 (1998-05-08), page 217 XP000857074
- VELDKAMP ET AL.: "A novel neutrophil-chemotaxis inhibitory protein of Staphylococcus aureus" ANTIMICROBIAL AGENTS AND CHEMOTHERAPY, vol. 38, 1998 - 27 September 1998 (1998-09-27), pages 24-27, XP000862866

## Description

The present invention relates to a new protein which can be used in the treatment of inflammation reactions. The invention further relates to a method of purifying the protein. Finally, the invention relates to a screening test with which analogous proteins can be detected.

An inflammation reaction is very generally a process wherein defence cells make their way to a source of infection and there ensure the elimination of the cause. Different mediators are herein released which contribute to elimination but also produce the inflammation symptoms. A distinction can be made between acute inflammations (such as sepsis) and latent chronic inflammations (such as rheumatism). In people with a lowered resistance acute inflammations can occur more often and more severely (as in the case of AIDS).

An infection with bacteria results in the formation of chemotactic factors which ensure that the leucocytes go to the source of the infection. A chemotactic substance is present in a gradient along which the leucocytes move in a directed manner. The source of a chemotactic agent can be the bacteria itself. These agents are for instance small proteins with a terminal formyl group, such as fMLP (N-formyl-Methionyl-Leucyl-Phenylalanine). Other chemotactic agents are activated complement factors (the anaphyloxins C3a and C5a), leukotrienes (such as LTB4 (Leukotriene B4) and PAF (Platelet-Activating Factor) and chemokines produced by different cell types such as interleukin-8 (monocytes and endothelial cells), RANTES (Regulated upon Activation, Normal T-cell Expressed and Secreted), eotaxin, MCP (Monocyte Chemotactic Protein), MIP (Macrophage Inflammatory Protein) and others.

Some chemokines are only specific to a determined type of leucocyte, others affect a plurality of cells. The receptors for chemokines are subdivided into two main groups, the CC and CXC receptors which all belong to the serpentines, receptors which traverse the membrane 7 times. The serpentines are rhodopsin-like GTP-binding protein linked receptors.

The super family of chemotactic cytokines, chemokines, is characterized by 4 conserved cysteines. Depending on the relative position of the first two cysteines, two families can be distinguished: the CXC or alpha-chemokines and the CC or beta-chemokines. The CXC chemokines are particularly active on granulocytes while the CC chemokines activate a wide range of leucocytes including monocytes, eosinophils, T-lymphocytes, NK cells and dendritic cells. This family of chemokines and also the classical chemotactic agents such as fMLP and C5a bind and activate the serpentines.

Neutralization of chemokines has already been applied experimentally, in particular the administering of antibodies against IL-8 was found to be effective in a number of animal experimental inflammation models. In addition, it has recently been demonstrated that a number of chemokine receptors (CCRS and CXCR4 in particular) play a part as co-factor in the infection of cells by HIV. For the T-cell-trophic strains of HIV, CXCR4 has been identified as co-factor and for monotrophic strains this is CCR5. Blocking of these receptors with antibodies or ligands inhibited the HIV infection in in vitro models. Moreover, people with a genetic variant of the CCR5 receptor consisting of a 32 base pair' deletion are found to be resistant to infection with HIV.

In addition to induction of chemotaxis, the directed migration of the leucocytes, in low concentrations a number of chemokines are also potent activators or primers of other leucocyte functions. It is therefore desirable to achieve a blocking of chemokines whereby inflammation reactions can be kept in check.

It is therefore the object of the present invention to provide a new agent with inflammation- inhibiting properties for the treatment of acute and chronic inflammation reactions and HIV.

During the research which led to the present invention a protein was found in the extracellular medium of growing Staphylococcus aureus (S. aureus) which was found capable of blocking different chemokine receptors. Incubation of different cells with the medium resulted in a greatly reduced expression of a number of the chemokine receptors, both of the expression of receptors of classical chemotactic agents such as fMLP and C5a on granulocytes and of the expression of CXCR4 and CCR5 receptors on lymphocytes, monocytes and macrophages. The reduced receptor expression was related to greatly reduced chemotaxis relative to the chemokines, as well as a reduced infection with HIV.

The activity of the protein is already manifest in the culture supernatant of the growing S.aureus. According to the invention however, the active protein was also purified by means of a number of Ligand Dye columns. A pre-purification was first performed on a so-called "yellow column" ("Reactive Yellow 86" ligand dye cross-linked 4% beaded agarose column (Sigma)), followed by an absorption chromatography column (the so-called "green column" ("Reactive Green 19" ligand dye cross-linked 4% beaded agarose column (Sigma)) and a DNA column (DNA Cellulose (Pharmacia)). Both latter columns can be interchanged. The DNA column removes a contaminant with the same molecular weight as the protein according to the invention. The absorption chromatography column concentrates the protein and is selective for the protein. Finally, a post-purification also takes place by means of gel filtration and optionally a concentration. In the gel filtration the protein with the molecular weight of about 17 kD is selected. This is the protein according to the invention.

Each step in the purification method can be monitored by means of testing the activity of the flow-through or the eluate of the different columns. This takes place by monitoring whether the flow-through or the eluate is able to prevent the binding of fMLP to granulocytes. An extensive test protocol is given in the examples.

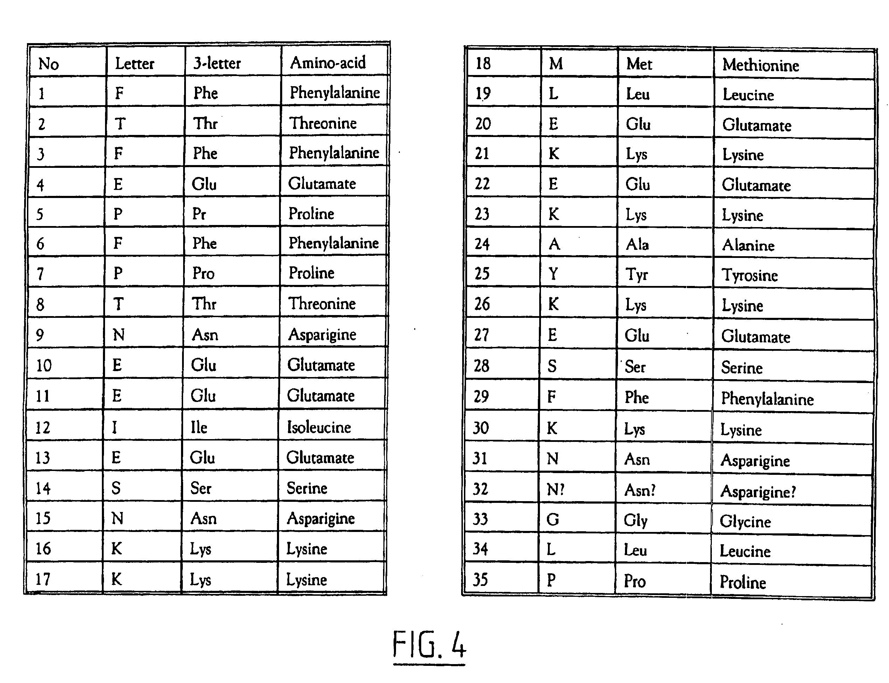

The first (N-terminal) 15 amino acids of the purified protein were determined by means of micro- sequencing. This sequence is given in figure 4. With sequence analysis no homology with any known bacterial or eucaryotic amino acid sequence was found in databases. This is therefore a new and unique protein.

Because this protein is isolated from the supernatant of the Staphylococcus aureus and gives inhibition of chemotaxis, this protein is herein also designated as "CHIPS": CHemotaxis Inhibitory Protein from Staphylococcus aureus.

The present invention therefore relates according to a first aspect thereof to the CHIPS protein, which is characterized by:
a) a molecular weight of about 17 kD;
b) the N-terminal amino acid sequence as given in figure 4; and
c) a biological activity which consists of the capacity to prevent the binding of fMLP to granulocytes in a test as described in example 1, and biologically active fragments thereof.

The CHIPS protein influences the chemotaxis of leucocytes to the source of the chemoattractant, such as the bacteria. It has been found according to the invention that the number of at least two receptors (fMLP and C5a) on the leucocytes, in particular granulocytes, is down-regulated. The down-regulation is reversible.

The invention further relates to a therapeutic composition, comprising a suitable excipient and the CHIPS protein and/or biologically active fragments thereof. The composition can be used for the treatment of acute and chronic inflammation reactions and HIV infection. The protein ensures that the receptors which provide movement of the leucocyte to the chemotactic substance are blocked.

The invention likewise relates to the CHIPS protein and/or biologically active fragments thereof for use in the treatment of acute and chronic inflammation reactions and HIV infection, as well as the use of the CHIPS protein and/or biologically active fragments thereof for the manufacture of a therapeutic preparation for the treatment of said symptoms.

According to a subsequent aspect of the invention antibodies against the CHIPS protein and/or fragments thereof are provided for use in the treatment of staphylococcus infection. The proteins will block the activity of CHIPS or its fragments and thus restart the chemotaxis terminated by the bacteria, whereby the natural defence against the bacteria is restored.

The therapeutic compositions, which according to the invention contain CHIPS or antibodies thereagainst as active ingredient, will be particularly intended for parenteral, and then specifically, intravenous use. The therapeutic compositions can be prepared by combining (i.e. mixing, dissolving etc.) CHIPS with pharmaceutically acceptable excipients suitable for intravenous administration. The concentration of the active ingredient in a therapeutic composition can vary between 0.001% and 100%, depending on the nature of the treatment and the method of administration. The dose of the active ingredient for administering likewise depends on the administering route and application, but may for instance vary between 0.001 and 1 mg per kg of body weight, preferably between 1 *µ*g and 100 *µ*g per kg of body weight.

The invention further relates to a method of purifying the CHIPS protein, comprising of:
a) guiding over an absorption chromatography column the culture supernatant of Staphylococcus aureus or a liquid obtained therefrom after pre-purification;
b1) subsequently guiding the flow-through of the absorption chromatography column first over an affinity chromatography column and thereafter guiding the eluate of the affinity chromatography column over a DNA column; or
b2) subsequently guiding the flow-through of the absorption chromatography column first over a DNA column and thereafter guiding the flow-through of the DNA column over an absorption chromatography column;
c) guiding the flow-through respectively the eluate of the last column of step b) over a gel filtration column and selecting the fraction with a molecular weight of about 17 kD.

"Flow-through" is herein understood to mean that part of the loaded liquid having situated therein the constituents which come from the column without extra treatment. The constituents in this flow-through do not bind to the column. "Eluate" is understood to mean the liquid which comes from the column after elution and which contains the constituents from the liquid loaded on the column which were bound to the column and were released again therefrom by the elution. In the method according to the invention the absorption column binds most constituents of the loaded culture medium or a liquid obtained therefrom after pre-purification. The affinity column binds CHIPS and the Snase (Staphylococcal Nuclease) which has the same molecular weight as CHIPS and the same affinity (or lack thereof) for the affinity column respectively the absorption column. The DNA column binds only the Snase, whereby this is separated from CHIPS.

This method works particularly well if the first affinity chromatography column is a so-called Ligand Dye "yellow" column, the second affinity chromatography column is a so-called Ligand Dye "green" column and the DNA column a DNA cellulose column.

Finally, the invention also relates to a determination or assay for determining the activity of the CHIPS protein or proteins with an analogous function, comprising of:
a) introducing into a first compartment labelled cells capable of chemotaxis, in particular leucocytes,
b) introducing one or more chemoattractants into a second compartment separated from the first compartment by a membrane permeable to at least the cells,
c) placing the protein for testing into the first compartment;
d) measuring the quantity of label in the second compartment after a determined time.,

The cells are capable of moving through the membrane in the direction of the chemoattractant. The presence of CHIPS or an analogous protein prevents the migration by deactivating the receptor(s) for the chemoattractant(s). This test can be applied more generally to also determine the chemotaxis-modulating activity of other substances. The method steps are then the same.

Proteins analogous to CHIPS which are found in this manner can be subjected to the same purification as CHIPS to thus be able to determine homology between the two.

The CHIPS protein according to the invention has also been found in S.epidermis as well as in S.aureus.

The manner in which CHIPS was isolated from the culture supernatant of Staphylococcus aureus via the steps of ligand dye affinity and absorption chromatography, gel filtration and concentration, in addition to the testing of the activity of CHIPS on different chemokine receptors on different leucocytes and the testing of the inhibition of HIV infection in T-cells and macrophages is described in the examples following hereinbelow, which are only intended by way of illustration and are not intended to limit the invention in any way whatsoever.

Reference is made in the examples to the following figures:
**Figure 1** shows the incubation of granulocytes with a concentration series of a Staphylococcus supernatant (SaS) and the effect on the fMLP receptors and the C5a receptors.
**Figure 2** shows the effect of a concentration series of SaS on the chemotaxis of granulocytes to fMLP.
**Figure 3** shows the fractions of the gel filtration column with the optical density (OD) (line with diamonds) and the activity in the fMLP receptor assay as a percentage of inhibition (bars).
**Figure 4** shows the sequence of the first 15 (N-terminal) amino acids of CHIPS (of the estimated 125 in total).
**Figure 5a** shows the incubation of granulocytes with a concentration series of purified CHIPS and the effect on the fMLP receptors and the C5a receptors.
**Figure 5b** shows the incubation of granulocytes with a concentration series of purified CHIPS and the effect on the directed migration to fMLP.
**Figure 6** shows the expression of CXCR4 on lymphocytes.

### EXAMPLES

### EXAMPLE 1

### Identification and isolation of CHIPS as chemotaxis-inhibiting molecule

### MATERIAL AND METHOD

### 1.1 Isolation of the protein

Staphylococcus aureus 1690 (a clinical isolate, Utrecht Teaching Hospital) or Staphylococcus aureus Newman (van Dr TJ Foster, Dublin) is cultured overnight in IMDM medium (Gibco) and subsequently diluted 1:40 in fresh IMDM for a 7 hour culture at 37°C. After pelleting of the bacteria the S. aureus supernatant (referred to as SaS) is collected, filtered over a 0.2 *µ*m filter and immediately used further. See also Veldkamp et al, Inflammation 21, 541-551 (1997).

A quantity of 2 litres of SaS is guided over three columns (25 ml) coupled in tandem. These three columns are successively a "Reactive Yellow 86" ligand dye cross-linked 4% beaded agarose column (Sigma), a DNA Cellulose (Pharmacia) and a "Reactive Green 19" ligand dye cross-linked 4% beaded agarose column (Sigma). After washing the green (Reactive Green 19) column is eluted with 2 M NaCl and the active fractions are pooled and concentrated 10x with polyethylene glycol. The concentrated material is separated on a Pharmacia Superdex-200 gel filtration column, whereafter the active fractions are pooled, concentrated, dialysed and freeze- dried. The final purified material is resuspended in a small volume of sterile water and used inter alia for micro-sequence analysis.

For this purpose a sample is analysed on a 12.5% SDS-PAGE (Mini-Protean II; BioRad) and transferred to Immobilon-P PVDF membrane (Millipore) by means of the Mini Trans-Blotter (BioRad). The proteins are stained with Coomassie Blue and the protein around 17 kD is excised. The N-terminal amino acid sequence of this sample is determined in accordance with the automated Edman procedure, wherein use is made of a Perkin Elmer/Applied Biosystems 476A. Amino acid derivatives are analysed by means of HPLC.

### 1.2 Binding of fMLP to granulocytes

Granulocytes are isolated from heparinized blood of healthy volunteers via a Histopaque-Ficoll gradient in accordance with the standard method (Troelstra et al, J. Leukocyte Biol. 61:173-178 (1997). The remaining erythrocytes in the granulocyte fraction are lysed with sterile water (for 30 sec) and washed after recovery of the isotonicity. The cells are finally resuspended in RPMI (Gibco) with 0.05% Human Serum Albumin (RPMI/HSA).

In Falcon tubes 50 *µ*l cells (5 x 10⁶ cells/ml) are incubated with 50 *µ*l CHIPS-containing material (SaS, purified CHIPS or column fractions) for 30 min at 37°C. The cells are placed on ice and washed once RPMI/HSA (at 4°C) and resuspended in 50 *µ*l fresh medium. 5 *µ*l BODIPY- labelled fMLP (end concentration 0.1 *µ*M; Molecular Probes) is then added and the sample is incubated for 60 minutes on ice. After washing the fluorescent fMLP binding to the granulocytes is analysed with a flowcytometer (FACScan; Becton Dickinson). The average fluorescence value of 5000 granulocytes is calculated with Lysis II software.

### 1.3 Chemotaxis

In order to determine the directed migration use is made of a Transwel system (Costar) consisting of an upper compartment and a lower compartment separated by a 3 *µ*m polycarbonate membrane. The granulocytes are labelled with BCECF (2-carboxyethyl-5-(and-6-) carboxyfluorescein; Molecular Probes), a fluorescent label which enters the cytoplasm of the cells. The cells (5x10⁶) are incubated for 20 minutes at 22°C with 3 *µ*M BCECF-AM (the acetomethyl ester of 2-carboxyethyl-5-(and-6-)-carboxyfluorescein), subsequently washed three times and resuspended to 5x10⁶ cells/ml in RPMI/HSA. 100 *µ*l of cells and the desired quantity of the CHIPS protein is introduced into the upper compartment of the Transwel system and the whole is suspended in the wells of a standard 24-well microtitre plate (Costar). Each well contains 600 *µ*l RPMI/HSA with or without addition of the chemoattractant for testing. The chemoattractants are: recombinant C5a (Sigma), recombinant interleukin-8 (Pepro Tech), Platelet Activating Factor-16 (PAF-16; Calbiochem) or fMLP (Sigma). After 60 minutes incubation at 37°C the Transwel container is lifted from the wells and the microtitre plate is analysed for fluorescence in a CyoFluorII (PerSeptiveBiosystems). The degree of fluorescence is a direct measure for the number of granulocytes which has migrated through the membrane and is expressed as a percentage of the fluorescence of the added number of cells.

### RESULTS

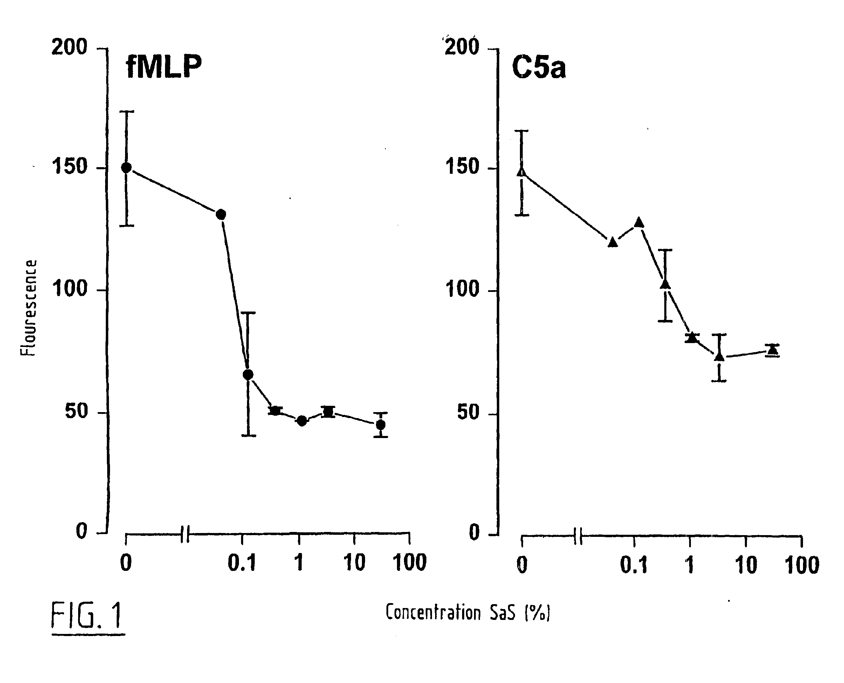

Figure 1 shows the effect of the incubation of granulocytes with a concentration series of a Staphylococcus-supernatant (SaS) on the fMLP receptors and the C5a receptors. A strong down regulation of both the C5A and the fMLP receptor is visible.

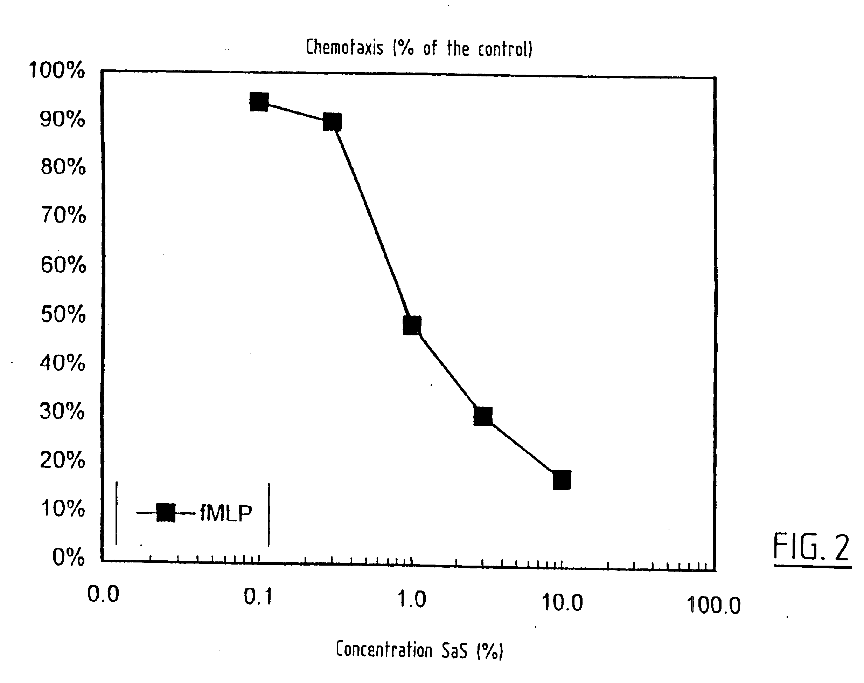

Figure 2 shows that the chemotaxis (cell movement) to the attractant (fMLP) is strongly inhibited.

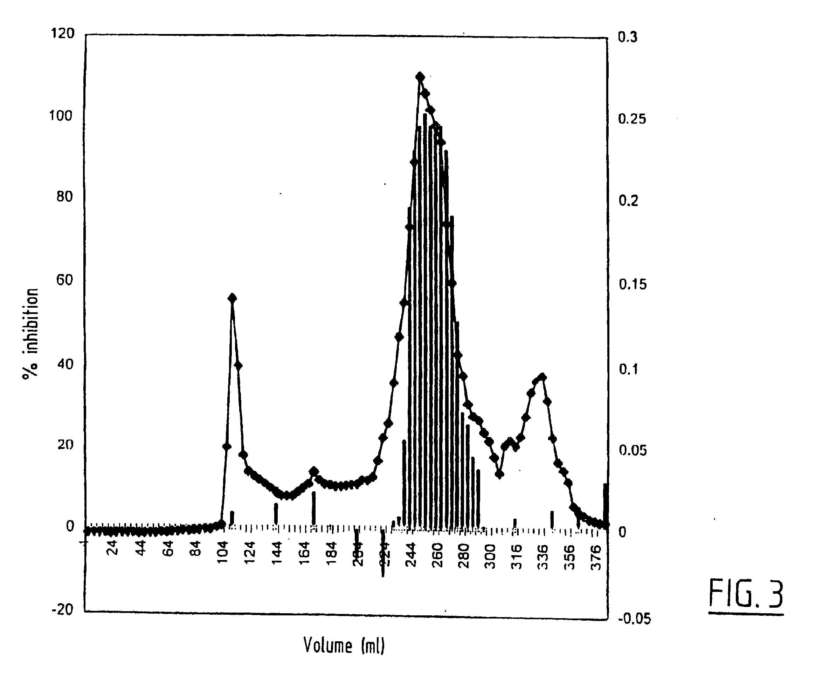

Figure 3 shows that the strongest inhibiting activity is situated in the elution range between 240 and 280 ml. The volume fractions correspond here with a protein of about 17 kD.

Figure 4 shows the sequence of the first 35 (N-terminal) amino acids of CHIPS (of the estimated 125 in total). On the basis of this sequence a synthetic peptide was made of the first 15 amino acids in accordance with standard Fmoc chemistry as described inter alia in De Haas et al, J.Immunol. 161:3607-3615 (1998) and Alonso de Velasco et al, Infect. Immun. 62:799-808 (1994). Antibodies generated against this peptide in rabbits (coupled to KLH in accordance with the instructions of the manufacturer, Pierce, and subcutaneously immunized with Freund's Complete Adjuvant, followed by a booster injection with Freund's Incomplete Adjuvant), as for instance described in Alonso de Velasco et al, supra, neutralize the activity of CHIPS.

### EXAMPLE 2

### Reduced expression of chemokine receptors on granulocytes due to CHIPS.

### MATERIAL AND METHOD

### 2.1 Receptor expression

The expression of the different chemokine receptors is determined with specific fluorescent- labelled antibodies and flowcytometry. The procedure followed is analogous to that described under 1.2 in example 1. Use is made of the following monoclonals: S5/1, anti-CD88 (C5a receptor) from Serotec Ltd; SE2, anti-CDw128A (IL-8 receptor) from Alexis Corporation; anti-PAF Receptor from Alexis Corporation. After incubation with CHIPS the cells are incubated for 30 min on ice with 5 *µ*g/ml antibody and after washing are labelled with a F(ab)2-FITC-labelled goat anti-murine Ig (Dako).

The average fluorescence of the granulocytes is a measure for the quantity of receptor on the cell surface. The relative expression, after subtraction of the background value, is expressed as a percentage of the cells which are incubated with control buffer.

### RESULTS

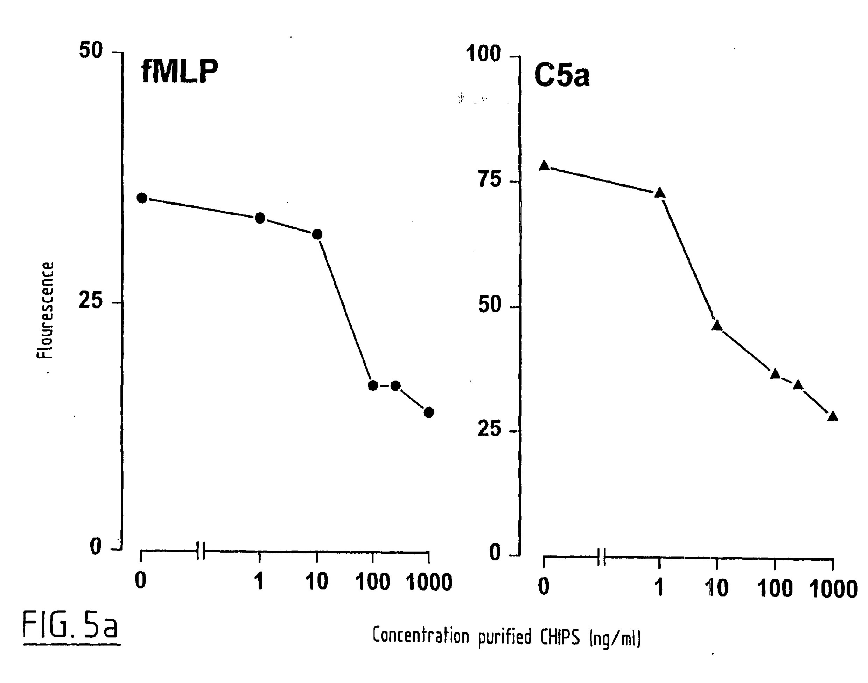

Figure 5a shows that both the C5a receptor and the fMLP receptor also disappear from the surface of the cells due to purified CHIPS.

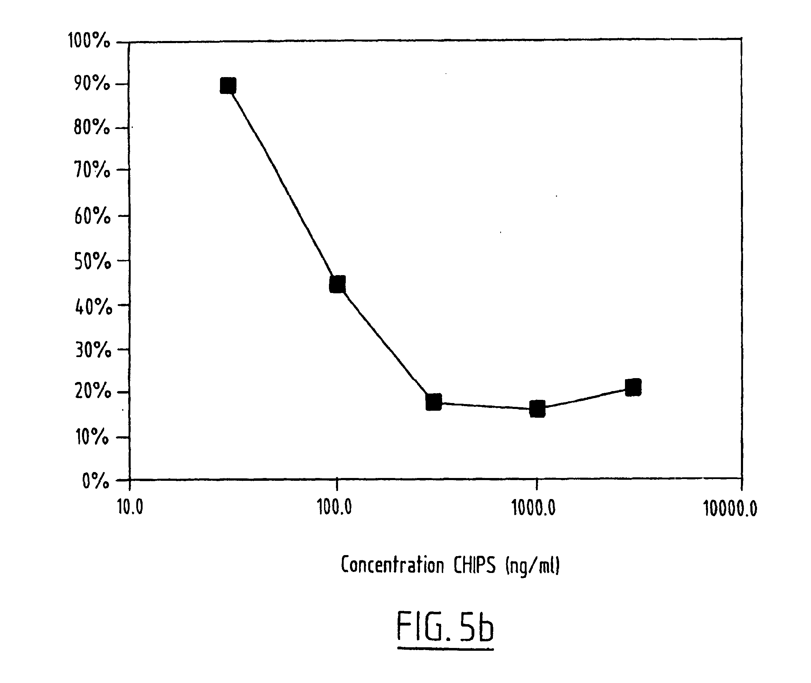

Figure 5b shows the incubation of granulocytes with a concentration series of purified CHIPS and the effect on the directed migration to fMLP. It can be seen that the chemotaxis (cell movement) to fMLP is inhibited completely and dose-dependently by purified CHIPS.

### EXAMPLE 3

### Reduced expression of chemokine receptors on T-cells due to CHIPS

### MATERIAL AND METHOD

### 3.1 Receptor expression

The mononuclear leucocytes (20% monocytes and 80% lymphocytes) are isolated from heparinized blood of healthy volunteers via a Ficoll gradient (Pharmacia) in accordance with the standard method (Antal-Szalmas et al, J. Leukocyte Biol. 61, 721-728 (1997). After washing the cells are resuspended in RPMI/HSA. The procedure followed for measuring expression of the different chemokine receptors is the same as described under 2.1 in example 2. To measure the expression of the lymphotrophic co-receptor for HIV (CXCR4) use is made of monoclonal 12G5 anti-CXCR4 from Becton Dickinson.

### RESULTS

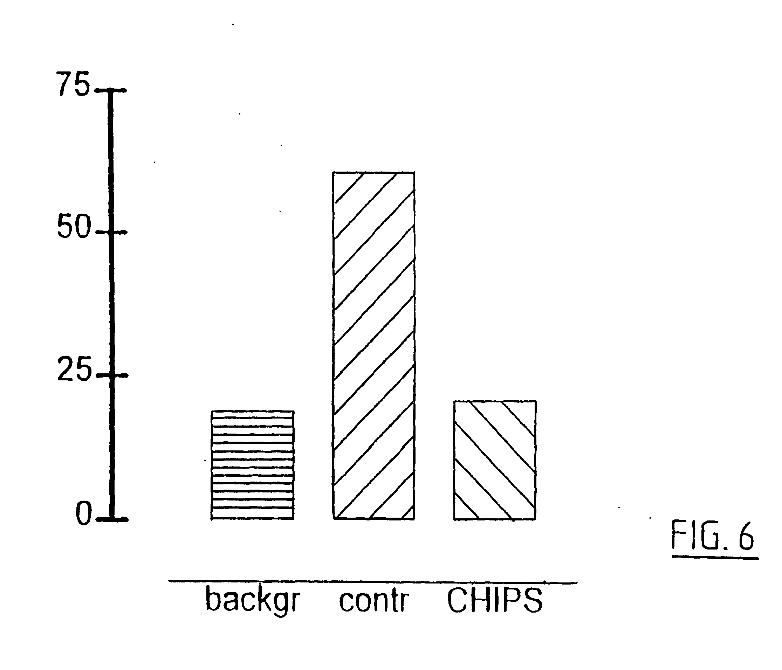

Figure 6 shows that after incubation of mononuclear cells with CHIPS, the expression of CXCR4 on lymphocytes disappears.

## Claims

1. Chemotaxis-inhibiting protein of Staphylococcus (CHIPS protein), which is **characterised by**:
a) a molecular weight of about 17 kD;
b) the N-terminal amino acid sequence as given in figure 4; and
c) a biological activity which consists of the capacity to prevent the binding of fMLP and/or C5a to granulocytes in a test as described in example 1, under 1.2, and fragments thereof that have the biological activity as defined under c).

2. The CHIPS protein and/or the biologically active fragments thereof as claimed in claim 1 for use as medicine.

3. The CHIPS protein and/or the biologically active fragments thereof as claimed in claim 1 for use in the treatment of acute and chronic inflammation reactions and HIV infection.

4. Use of the CHIPS protein and/or the biologically active fragments thereof as claimed in Claim 1 for the manufacture of a therapeutic preparation for the treatment of acute and chronic inflammation reactions and HIV infection.

5. Antibodies against the CHIPS protein and/or the biologically active fragments thereof as claimed in Claim 1.

6. Antibodies as claimed in Claim 5 for use in the treatment of staphylococcus infection.

7. Therapeutic composition comprising a suitable excipient and the CHIPS protein and/or the biologically active fragments thereof as claimed in Claim 1.

8. Composition as claimed in Claim 7 for treating acute and chronic inflammation reactions and HIV infection.

9. Therapeutic composition comprising a suitable excipient and one or more antibodies against the CHIPS protein and/or the biologically active fragments thereof as claimed in Claim 1.

10. Method of purifying the CHIPS protein as claimed in Claim 1, comprising the steps of:
a) guiding over an absorption chromatography column the culture supernatant of Staphylococcus aureus or a liquid obtained therefrom after pre-purification;
b1) subsequently guiding the flow-through of the absorption chromatography column first over an affinity chromatography column and thereafter guiding the eluate of the affinity chromatography column over a DNA column; or
b2) subsequently guiding the flow-through of the absorption chromatography column first over a DNA column and thereafter guiding the flow-through of the DNA column over an absorption chromatography column;
c) guiding the flow-through of the last column of step b1) respectively the eluate of the last column of step b2) over a gel filtration column and selecting the fraction with a molecular weight of about 17 kD.

11. Method as claimed in Claim 10, **characterised in that** the affinity chromatography column is a so-called Ligand Dye "yellow" column, the absorption chromatography column is a so-called Ligand Dye "green" column and the DNA column a DNA cellulose column.

12. Method of determining the activity of the CHIPS protein and/or the biologically active fragments thereof as claimed in Claim 1, comprising the steps of :
a) introducing into a first compartment labelled cells capable of chemotaxis, in particular leukocytes,
b) introducing one or more chemoattractants into a second compartment separated from the first compartment by a membrane permeable to at least the cells,
c) placing the protein for testing into the first compartment,
d) measuring the quantity of label in the second compartment after a determined time.

13. Method of determining the chemotaxis-modulating activity of the CHIPS protein and/or the biologicaly active fragments these of as claimed in Clm 1 comprising the steps of:
a) introducing into a first compartment labelled cells capable of chemotaxis, in particular leukocytes.
b) introducing one or more chemoattractants into a second compartment separated from the first compartment by a membrane permeable to at least the cells,
c) placing the substance for testing into the first compartment.
d) measuring the quantity of label in the second compartment after a determined time.

14. Method of determining binding of fMLP to granulocytes in the presence of CHIPS-containing material comprising the steps of:
a) incubating granulocytes suspended in RPMI medium with 0.05% Human Serum Albumin (RPMI/HSA) with CHIPS-containing material for 30 min. at 37°C;
b) placing the granulocytes on ice and washing them once in RPMI/HSA at 4°C;
c) resuspending the granulocytes in fresh RPMI/HSA medium;
d) incubating the granulocytes with fluorescently labeled fMLP in order to effect binding of the labeled fMLP to the granulocytes;
e) washing away the unbound fluorescent label; and
f) analysing the binding of the fluorescent fMLP to the granulocytes by measuring the fluorescence.

## Patentansprüche

1. Chemotaxis inhibierendes Protein von Staphyloccocus (CHIPS-Protein), das **gekennzeichnet ist durch**:
a) ein Molekulargewicht von ungefähr 17 kD;
b) die N-terminale Aminosäuresequenz, die in Fig. 4 angegeben ist; und
c) eine biologische Aktivität, die aus der Kapazität besteht, das Binden von fMLP und/oder C5a an Granolozyten in einem Test zu verhindern, wie er in Beispiel 1 unter 1.2 beschrieben ist,
und Fragmente davon, die die biologische Aktivität aufweisen, wie sie unter c) definiert ist.

2. CHIPS-Protein und/oder biologisch aktive Fragmente davon, wie sie in Anspruch 1 beansprucht sind, zur Verwendung als Medikament.

3. CHIPS-Protein und/oder biologisch aktive Fragmente davon, wie sie in Anspruch 1 beansprucht sind, zur Verwendung bei der Behandlung von akuten oder chronischen entzündlichen Reaktionen und HIV-Infektion.

4. Verwendung des CHIPS-Proteins und/oder der biologisch aktiven Fragments davon, wie sie in Anspruch 1 beansprucht sind, für die Herstellung einer therapeutischen Präparation zur Behandlung von akuten und chronischen entzündlichen Reaktionen und HIV-Infektion.

5. Antikörper gegen das CHIPS-Protein und/oder die biologisch aktiven Fragmente davon, wie sie in Anspruch 1 beansprucht sind.

6. Antikörper, wie er in Anspruch 5 beansprucht ist, zur Verwendung bei der Behandlung von Staphyloccosus-Infektion.

7. Therapeutische Zusammensetzung, die ein geeignetes Vehikel und das CHIPS-Protein und/oder die biologisch aktiven Fragmente davon enthält, wie sie in Anspruch 1 beansprucht sind.

8. Zusammensetzung, wie sie in Anspruch 7 beansprucht ist, zur Behandlung von akuten und chronischen entzündlichen Reaktionen und HIV-Infektion.

9. Therapeutische Zusammensetzung, die ein geeignetes Vehikel und einen oder mehrere Antikörper gegen das CHIPS-Protein und/oder die biologisch aktiven Fragmente davon aufweist, wie sie in Anspruch 1 beansprucht sind.

10. Verfahren zum Reinigen des CHIPS-Proteins, wie es in Anspruch 1 beansprucht ist, mit den Schritten:
a) Führen des Kulturüberstands von Staphyloccocus aureus oder einer davon nach Vorreinigung erhaltenen Flüssigkeit über eine Absorptionschromatographiesäule;
b1) nachfolgendes Führen des Durchflusses der Absorptionschromatographiesäule zunächst über eine Affinitätschromatographiesäule und danach Führen des Eluats der Affinitätschromatographiesäule über eine DNS-Säule; oder
b2) nachfolgendes Führen des Durchflusses der Absorptionschromatographiesäule erst über eine DNS-Säule und danach Führen des Durchflusses der DNS-Säule über eine Absorptionschromatographiesäule;
c) Führen des Durchflusses der letzten Säule von Schritt b1) bzw. des Eluats von der letzten Säule des Schritts b2) über eine Gelfiltrationssäule und Selektieren der Fraktion mit einem Molekulargewicht von ungefähr 17 kDa.

11. Verfahren wie in Anspruch 10 beansprucht, **dadurch gekennzeichnet, dass** die Affinitätschromatographiesäule eine so genannte Ligandenfarbstoff "Gelb"-Säule ist, die Absorptionschromatographiesäule eine so genannte Ligandenfarbstoff "Grün"-Säule ist und die DNS-Säule eine DNS-Cellulosesäule ist.

12. Verfahren zum Bestimmen der Aktivität des CHIPS-Proteins und/oder der biologisch aktiven Fragmente davon, wie sie in Anspruch 1 beansprucht sind, mit den Schritten:
a) Einführen von markierten Zellen, die zur Chemotaxis in der Lage sind, insbesondere-Leukozyten, in ein erstes Abteil;
b) Einführen eines oder mehrerer chemischer Anziehungsstoffe in ein zweites Abteil, das von dem ersten Abteil durch eine Membran getrennt ist, die zumindest für die Zellen permeabel ist;
c) Anordnen des Proteins zum Testen in das erste Abteil,
d) Messen der Menge an Markierung in dem zweiten Abteil nach einer vorgegebenen Zeit.

13. Verfahren zum Bestimmen der Chemotaxis-modulierenden Aktivität des CHIPS-Proteins und/oder der biologisch aktiven Fragmente davon, wie sie in Anspruch 1 beansprucht sind, dass die Schritte aufweist:
a) Einführen von markierten Zellen, die zu Chemotaxis in der Lage sind, insbesondere Leukozyten, in ein erstes Abteil,
b) Einführen eines oder mehrerer chemischer Anziehungsmittel in ein zweites Abteil, das von dem ersten Abteil durch eine Membran getrennt ist, die zumindest für die Zellen permeabel ist;
c) Anordnen der Substanz zum Testen in das erste Abteil,
d) Messen der Menge an Markierung in dem zweiten Abteil nach einer vorgegebenen Zeit.

14. Verfahren zum Bestimmen der Bindung von fMLP an Granulozyten in der Anwesenheit von CHIPS-enthaltendem Material, mit den Schritten:
a) Inkubieren von Granulozyten, die in RPMI-Medium mit 0,05 % humanem Serumalbumin (RPMI/HSA) suspendiert sind, mit CHIPS-enthaltendem Material für 30 min bei 37 °C;
b) Anordnen der Granulozyten auf Eis und einmal Waschen der Granulozyten in RPMI/HSA bei 4 °C;
c) erneutes Suspendieren der Granulotzyten in frischem RPMI/HSA-Medium;
d) Inkubieren der Granolozyten mit fluoreszent markiertem fMLP, um die Bindung des markierten fMLP an die Granulozyten zu bewirken;
e) Wegwaschen der ungebundenen Fluoreszenzmarkierung; und
f) Analysieren der Bindung des fluoreszenten fMLP an die Granulozyten durch Messen der Fluoreszenz.

## Revendications

1. Protéine inhibant la chimiotaxie de *Staphylococcus* (protéine CHIPS), qui est **caractérisée par** :
a) un poids moléculaire d'environ 17 kD ;
b) une séquence d'acides aminés N-terminale telle que présentée à la figure 4 ; et
c) une activité biologique qui consiste en la capacité à prévenir la liaison de fMLP et/ou de C5a à des granulocytes dans un essai tel que décrit dans l'exemple 1, en 1.2,
et des fragments de celle-ci qui ont l'activité biologique telle que définie en c).

2. Protéine CHIPS et/ou les fragments biologiquement actifs de celle-ci tels que revendiqués dans la revendication 1, pour l'utilisation en tant que médicament.

3. Protéine CHIPS et/ou les fragments biologiquement actifs de celle-ci tels que revendiqués dans la revendication 1, pour l'utilisation dans le traitement de réactions d'inflammation aiguë et chronique et d'une infection par le VIH.

4. Utilisation de la protéine CHIPS et/ou les fragments biologiquement actifs de celle-ci tels que revendiqués dans la revendication 1, pour la fabrication d'une préparation thérapeutique pour le traitement de réactions d'inflammation aiguë et chronique et d'une infection par le VIH.

5. Anticorps dirigés contre la protéine CHIPS et/ou les fragments biologiquement actifs de celle-ci tels que revendiqués dans la revendication 1.

6. Anticorps selon la revendication 5, pour l'utilisation dans le traitement d'une infection à *Staphylococcus.*

7. Composition thérapeutique comprenant un excipient approprié et la protéine CHIPS et/ou les fragments biologiquement actifs de celle-ci tels que revendiqués dans la revendication 1.

8. Composition selon la revendication 7, pour traiter des réactions d'inflammation aiguë et chronique et une infection par le VIH.

9. Composition thérapeutique comprenant un excipient approprié et un ou plusieurs anticorps dirigés contre la protéine CHIPS et/ou les fragments biologiquement actifs de celle-ci tels que revendiqués dans la revendication 1.

10. Procédé de purification de la protéine CHIPS telle que revendiquée dans la revendication 1, comprenant les étapes consistant :
a) à guider au-dessus d'une colonne de chromatographie d'absorption le surnageant de culture de *Staphylococcus aureus* ou un liquide obtenu à partir de celui-ci après pré-purification ;
b1) puis à guider le produit d'écoulement de la colonne de chromatographie d'absorption d'abord sur une colonne de chromatographie par affinité puis à guider l'éluat de la colonne de chromatographie par affinité sur une colonne d'ADN ; ou
b2) puis à guider le produit d'écoulement de la colonne de chromatographie d'absorption d'abord sur une colonne d'ADN puis à guider le produit d'écoulement de la colonne d'ADN sur une colonne de chromatographie d'absorption ;
c) à guider le produit d'écoulement de la dernière colonne de l'étape b1), respectivement l'éluat de la dernière colonne de l'étape b2), sur une colonne de filtration sur gel et à choisir la fraction ayant un poids moléculaire d'environ 17 kD.

11. Procédé selon la revendication 10, **caractérisé en ce que** la colonne de chromatographie par affinité est une colonne dite à colorant ligand "jaune", la colonne de chromatographie d'absorption est une colonne dite à colorant ligand "vert" et la colonne d'ADN une colonne d'ADN cellulose.

12. Procédé de détermination de l'activitë de la protéine CHIPS et/ou des fragments biologiquement actifs de celle-ci tels que revendiqués dans la revendication 1, comprenant les étapes consistant :
a) à introduire dans un premier compartiment des cellules marquées capables de chimiotaxie, en particulier des leucocytes,
b) à introduire un ou plusieurs agents de chimio-attraction dans un second compartiment séparé du premier compartiment par une membrane perméable aux moins aux cellules,
c) à placer la protéine à tester dans le premier compartiment,
d) à mesurer la quantité de marqueur dans le second compartiment après un temps déterminé.

13. Procédé de détermination de l'activité de modulation de chimiotaxie de la protéine CHIPS et/ou des fragments biologiquement actifs de celle-ci tels que revendiqués dans la revendication 1, comprenant les étapes consistant :
a) à introduire dans un premier compartiment des cellules marquées capables de chimiotaxie, en particulier des leucocytes,
b) à introduire un ou plusieurs agents de chimio-attraction dans un second compartiment séparé du premier compartiment par une membrane perméable au moins aux cellules,
c) à placer la substance à tester dans le premier compartiment,
d) à mesurer la quantité de marqueur dans le second compartiment après un temps déterminé.

14. Procédé de détermination de la liaison de fMLP à des granulocytes en présence de matériau contenant CHIPS, comprenant les étapes consistant :
a) à incuber des granulocytes en suspension dans du milieu RPMI avec 0,05 % de sérumalbumine humaine (RPMI/HSA) avec un matériau contenant CHIPS pendant 30 min. à 37 °C ;
b) à mettre les granulocytes sur de la glace et à les laver une fois dans du RMPI/HSA à 4 °C ;
c) à remettre en suspension les granulocytes dans du milieu RPMI/HSA frais ;
d) à incuber les granulocytes avec du fMLP marqué par fluorescence afin d'effectuer une liaison du fMLP marqué aux granulocytes ;
e) à enlever par lavage le marqueur fluorescent non lié ; et
f) à analyser la liaison du fMLP fluorescent aux granulocytes en mesurant la fluorescence.
